(19) Europäisches Patentamt
European Patent Office
Office européen des brevets

(11) Publication number: **0 246 888**
**A2**

(12) **EUROPEAN PATENT APPLICATION**

(21) Application number: **87304504.1**

(22) Date of filing: **20.05.87**

(51) Int. Cl.4: **C 07 D 249/12**
A 61 K 31/41, C 07 C 133/02,
C 07 C 159/00

(30) Priority: **23.05.86 US 866437**

(43) Date of publication of application:
**25.11.87 Bulletin 87/48**

(84) Designated Contracting States:
**AT BE CH DE ES FR GB GR IT LI LU NL SE**

(71) Applicant: **SMITHKLINE BECKMAN CORPORATION**
**One Franklin Plaza P O Box 7929**
**Philadelphia Pennsylvania 19103 (US)**

(72) Inventor: **Finkelstein, Joseph Alan**
**7549 Brentwood Road**
**Philadelphia Pennsylvania 19151 (US)**

**Kruse, Lawrence Ivan**
**33 Firs Walk**
**Tewin Hertfordshire AL6 0NY (GB)**

(74) Representative: **Giddings, Peter John, Dr. et al**
**Smith Kline & French Laboratories Ltd. Corporate Patents Mundells**
**Welwyn Garden City Hertfordshire AL7 1EY (GB)**

(54) **Dopamine-beta-hydroxylase inhibitors.**

(57) Compounds of Formula :

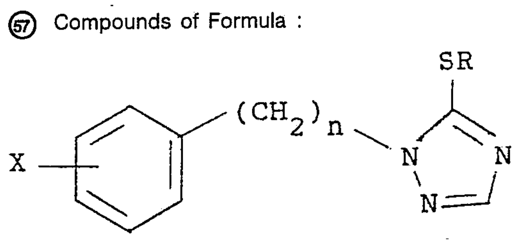

in which n is 0 to 5, R is hydrogen or $C_{1-4}$alkyl and X is hydrogen, halo, $C_{1-4}$alkyl, CN, $NO_2$, $SO_2NH_2$, COOH, CHO, OH, $CH_2OH$, $C_{1-4}$alkoxy, $CF_3$, $SO_2CH_3$, $SO_2CF_3$ or $CO_2C_aH_{2a+1}$wherein a is 1 to 5 or any synthetically accessible combination thereof of up to 5 substituents or any pharmaceutically acceptable salt or hydrate thereof, processes for their preparation, pharmaceutical compositions containing them and their use in therapy as dopamine β-hydroxylase inhibitors.

EP 0 246 888 A2

**Description**

DOPAMINE-β-HYDROXYLASE INHIBITORS

FIELD OF THE INVENTION

This invention relates to novel compounds that inhibit dopamine-β-hydroxylase.

BACKGROUND OF THE INVENTION

In the catecholamine biosynthetic pathway, tyrosine is converted in three steps to norepinephrine (NE). Intermediates are dihydroxyphenylalanine (DOPA) and dopamine (DA). Dopamine is hydroxylated to norepinephrine by dopamine-β-hydroxylase (DBH) in the presence of oxygen and ascorbic acid.

Inhibition of catecholamine activity decreases blood pressure. Weinshilboum, Mayo Clin. Proc. 55, 39 (1980), reviews compounds that inhibit catecholamine activity by acting upon adrenergic receptors. Alternatively, the catecholamine biosynthetic pathway can be suppressed at any of the three steps, resulting in reduced NE levels. In addition to producing an antihypertensive effect, inhibitors of NE synthesis are active as diuretics, natriuretics, cardiotonics, and vasodilators. Inhibition of DBH activity can have the added advantage of increasing DA levels, which as reported by Ehrreich et al., "New Antihypertensive Drugs," Spectrum Publishing, 1976, pp. 409-432, has selective vasodilator activity at certain concentrations.

DBH inhibitors also have been shown to reduce or prevent formation of gastric ulcers in rats by Hidaka et al., "Catecholamine and Stress," edit. by Usdin et al., Permagon Press, Oxford, 1976, pp. 159-165 and by Osumi et al., Japan J. Pharmacol. 23, 904 (1973).

A number of DBH inhibitors are known. These generally are divided into two classes, namely, metal chelating agents, which bind copper in the enzyme, and phenethylalamine analogues. Rosenberg et al., "Essays in Neurochemistry and Neuropharmacology," Vol. 4, ed. by Youdim et al., John Wiley & Sons, 1980, pp. 179-192, and Goldstein, Pharmacol. Rev. 18(1), 77 (1966), review DBH inhibitors. The former report that many potent DBH inhibitors have a hydrophobic side chain of size comparable to the aromatic ring of DA, leading the authors to suggest that incorporation of a terminal hydroxyl group on a 4- to 6- carbon side chain on a phenethylalamine analogue may yield potent inhibitors.

Known DBH inhibitors include:

(a) 5-alkylpicolinic acids [See, Suda et al., Chem. Pharm. Bull. 17, 2377 (1969) ; Umezawa et al., Biochem. Pharmacol. 19, 35 (1969) ; Hidaka et al., Mol. Pharmacol. 9, 172 (1973) ; Miyano et al., Chem. Pharm. Bull. 26, 2328 (1978) ; Miyano et al., Heterocycles 14, 755 (1980) ; Claxton et al., Eur. J. Pharmacol. 37, 179 (1976)] ;

(b) BRL 8242 [See, Claxton et al., Eur. J. Pharmacol. 37, 179 (1976)] ;

(c) 1-alkylimidazole-2-thiols [See, Hanlon et al., Life Sci. 12, 417 (1973) ; Fuller et al., Adv. Enzyme Regul. 15, 267 (1976)] ;

(d) substituted thioureas [See, Johnson et al., J. Pharmacol. Exp. Ther. 168, 229 (1969) ] ; and

(e) benzyloxyamine and benzylhydrazine [See, Creveling et al., Biochim. Biophys. Acta 64, 125 (1962) ; Creveling et al., Biochim. Biophys. Acta 8, 215 (1962) ; Van Der Schoot et al., J. Pharmacol. Exp. Ther. 141, 74 (1963) ; Bloom, Ann. N.Y. Acad. Sci 107, 878 (1963) ].

All the above compounds except benzyloxyamine and benzylhydrazine apparently owe their inhibitory effect to metal chelating properties. Alkyl derivatives of imidazole-2-thiol are more potent, presumably due to non-specific interaction of the alkyl substituent with the enzyme. Benzyloxyamine and benzylhydrazine are phenethylalamine analogues which apparently act as competitive inhibitors.

In addition to the above compounds, Runti et al., Il Farmaco Ed. Sci. 36, 260 (1980), report that other fusaric acid derivatives and analogues inhibit DBH. These include phenylpicolinic acid, which has twice the inhibitory activity of fusaric acid, and 5-(4-chlorobutyl) picolinic acid, and others such as substituted amides of fusaric acid and acids and amides of 5-butyroylpicolinic acid, 5-aminopicolinic acid and 5-hydrazinopicolinic acid, and derivatives thereof.

Hidaka et al., Molecular Pharmacology, 9, 172-177 (1972) report that 5-(3,4-dibromobutyl)picolinic acid and 5-(dimethyldithiocarbamoylmethyl)picolinic acid are DBH inhibitors.

Bupicomide, 5-(n-butyl)picolinamine, is reported by Ehrreich et al., "New Antihypertensive Drugs", Spectrum Publications, 1976, pg. 409-432, to be a DBH inhibitor that has antihypertensive activity.

In European Patent Application No. 125,033 (published November 14, 1984) a series of 1-phenyl and 1-phenylalkylimidazole compounds having a mercapto or alkylthio group in the 2-position are disclosed. These compounds are described as having DBH inhibiting activity.

United States Patent No. 4,487,761 describes several methylpyridine derivatives isolated from the fermentation broth of a strain of Streptoverticillium. These compounds inhibit DBH activity.

United States Patent No. 4,532,331 describes various 1-benzyl-2-aminomethyl imidazole derivatives that inhibit DBH activity and includes pharmaceutical compositions containing these derivatives and methods of using these derivatives to inhibit DBH activity.

Non-specific, often toxic effects of known DBH inhibitors have obviated clinical use of these compounds. Fusaric acid, for example, is hepatotoxic. See, for example, Teresawa et al., Japan. Cir. J. 35, 339 (1971) and references cited therein. Presumably, the picolinic acid structure interacts with a number of metalloproteins

and enzymes non-specifically to produce the observed side effects.

4-Aralkyl substituted-1,2,4-triazole-3-thiols having the following structure have been synthesized previously

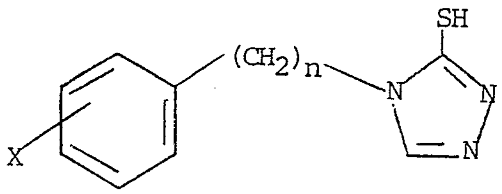

in which:
n is 0 and X is hydrogen, $CH_3$, $OCH_3$, $OCH_2CH_3$, $OCH_2CH_2CH(CH_3)_2$, Br, Cl, I, $CF_3$, $NO_2$, or COOH, or combinations of the above; and
n is 1 and X is hydrogen.
See, e.g., Chem. Abstr. 76:135847w; Chem. Abstr. 88:170043; Chem. Abstr. 67:38283; Chem. Abstr. 94:65563; Chem. Abstr. 84:44070; Chem. Abstr. 73:36594; Chem. Abstr. 89:215405.

Certain 1-aralkyl-substituted-1,2,4-triazole-5-thiols also have been prepared previously. These known compounds include compounds having the following formula:

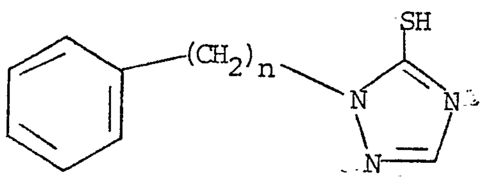

in which n is 0 or 1. See, e.g., Chem. Abstr. 74:99310; Chem. Abstr. 74:111161; Chem. Abstr. 75:146214; Chem. Abstr. 70:37729; Chem. Abstr. 79:66362; Chem. Abstr. 80:54530.

Absent from the above references disclosing the 4-aralkyl-substituted-1,2,4-triazole-3-thiol and 1-aralkyl-substituted-1,2,4-triazole-5-thiol compounds, however, is any suggestion that these compounds possess activity as dopamine-β-hydroxylase inhibitors or are efficacious in the treatment of diseases, such as hypertension, in which reductions in dopamine-β-hydroxylase activity produce therapeutic benefits. These compounds have been employed as reagents in photographic and electrorecording processes and analytical methods. Also, some of these compounds have been used as fungicides, herbicides, and pesticides. Additionally, certain of these compounds were found to inhibit the growth of mice footpads of leprosy-causing bacteria.

## SUMMARY OF THE INVENTION

The present invention resides in the discovery that DBH is inhibited by substituted 1-aralkyl-1,2,4-triazole-5-thiol and substituted 1-aralkyl-1,2,4-triazole-5-alkylthiol compounds. These compounds are potent and produce prolonged DBH inhibition.

Presently preferred compounds of the invention and compounds included in the pharmaceutical compositions and methods of the invention include:
1-benzyl-1,2,4-triazole-5-thiol;
1-(3′-fluorobenzyl)-1,2,4,triazole-5-thiol; and
1-(3′,5′-difluorobenzyl)-1,2,4-triazole-5-thiol.

In a further aspect of the invention there are provided novel intermediates useful in preparing substituted 1-aralkyl-1,2,4-triazole-5-thiol and substituted 1-aralkyl-1,2,4-triazole-5-alkylthiol compounds.

The invention includes novel processes for preparing intermediates useful in preparing substituted 1-aralkyl-1,2,4-triazole-5-thiol and substituted 1-aralkyl-1,2,4-triazole-5-alkylthiol compounds.

The invention also is compounds for use in therapy, particularly in inhibiting DBH activity in mammals, including humans.

Included in the present invention are pharmaceutical compositions comprising compounds useful in the method of the invention and a pharmaceutical carrier.

## DETAILED DESCRIPTION OF THE INVENTION

The presently invented compounds that inhibit DBH have the following formula:

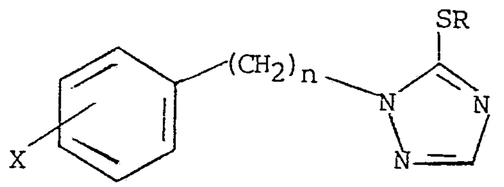

(I)

in which:
n is 0-5;
R is hydrogen or $C_{1-4}$ alkyl; and
X is hydrogen, halo, $C_{1-4}$ alkyl, CN, $NO_2$, $SO_2NH_2$, COOH, CHO, OH, $CH_2OH$, $C_{1-4}$ alkoxy, $SO_2CH_3$, $SO_2CF_3$, or $CO_2C_aH_{2a+1}$ wherein a is 1-5, or any synthetically accessible combination thereof of up to 5 substituents; or any pharmaceutically acceptable salt or hydrate thereof; except compounds in which n is 0 or 1 and X is hydrogen.

As used in the specification and claims, halo means bromo, fluoro, chloro, or iodo.

Suitably R is $C_{1-4}$ alkyl, preferably R is hydrogen. Suitably n is 0, 2, 3, or 4, preferably n is 1. Suitably X is a synthetically accessible combination of two substituents, preferably X is one substituent.

Included in the pharmaceutical compositions of the present invention and employed in the methods of the present invention are compounds having the following formula:

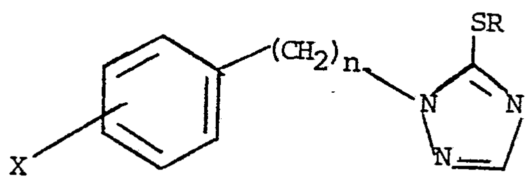

(II)

in which:
n is 0-5;
R is hydrogen or $C_{1-4}$ alkyl; and
X is hydrogen, halo, $C_{1-4}$ alkyl, CN, $NO_2$, $SO_2NH_2$, COOH, CHO, OH, $CH_2OH$, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents; or
any pharmaceutically acceptable salt or hydrate thereof.

It is intended that Formulae I and II include the tautomer of the compounds in which R is hydrogen, that is, compounds having the above formulae wherein the triazole moiety has either of the below formulae:

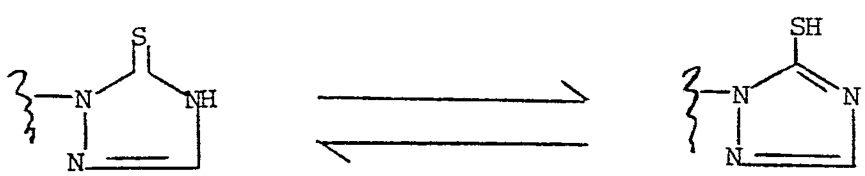

The compounds of Formulae I and II in which n is 1 are prepared from corresponding benzaldehydes by processes such as shown in Scheme I, below. The starting benzaldehydes are known and described in published references or can be obtained readily.

Scheme I illustrates reaction of benzaldehydes (A) having X substituents, which are the same as X in Formula I, with t-butylcarbazate in an organic solvent such as hexane to produce substituted t-butoxycarbonylhydrazones (B).

Substituted t-butoxycarbonylhydrazines (C) then are prepared by hydrogenation of the substituted t-butoxycarbonylhydrazones (B) using a hydogenating agent such as palladium, preferably 10%, on carbon in an inert organic solvent such as ethanol, methylene chloride, chloroform, tetrahydrofuran, ethyl acetate, or ethyl ether, preferably methanol.

Then the t-butoxycarbonylhydrazines (C) are reacted with t-butylisothiocyanate in an inert organic solvent, preferably ethyl acetate, to produce 2-substituted-1-t-butoxycarbonyl-4-t-butylthiosemicarbazide compounds

(D).

The compounds of Formulae (I) and (II) wherein R is hydrogen then are prepared by cyclization of the 2-substituted-1-t-butoxycarbonyl-4-t-butylthiosemicarbazides (D) in the presence of strong acid such as hydrochloric, hydrobromic, acetic, trifluoroacetic, or propionic, preferably formic acid.

Scheme I

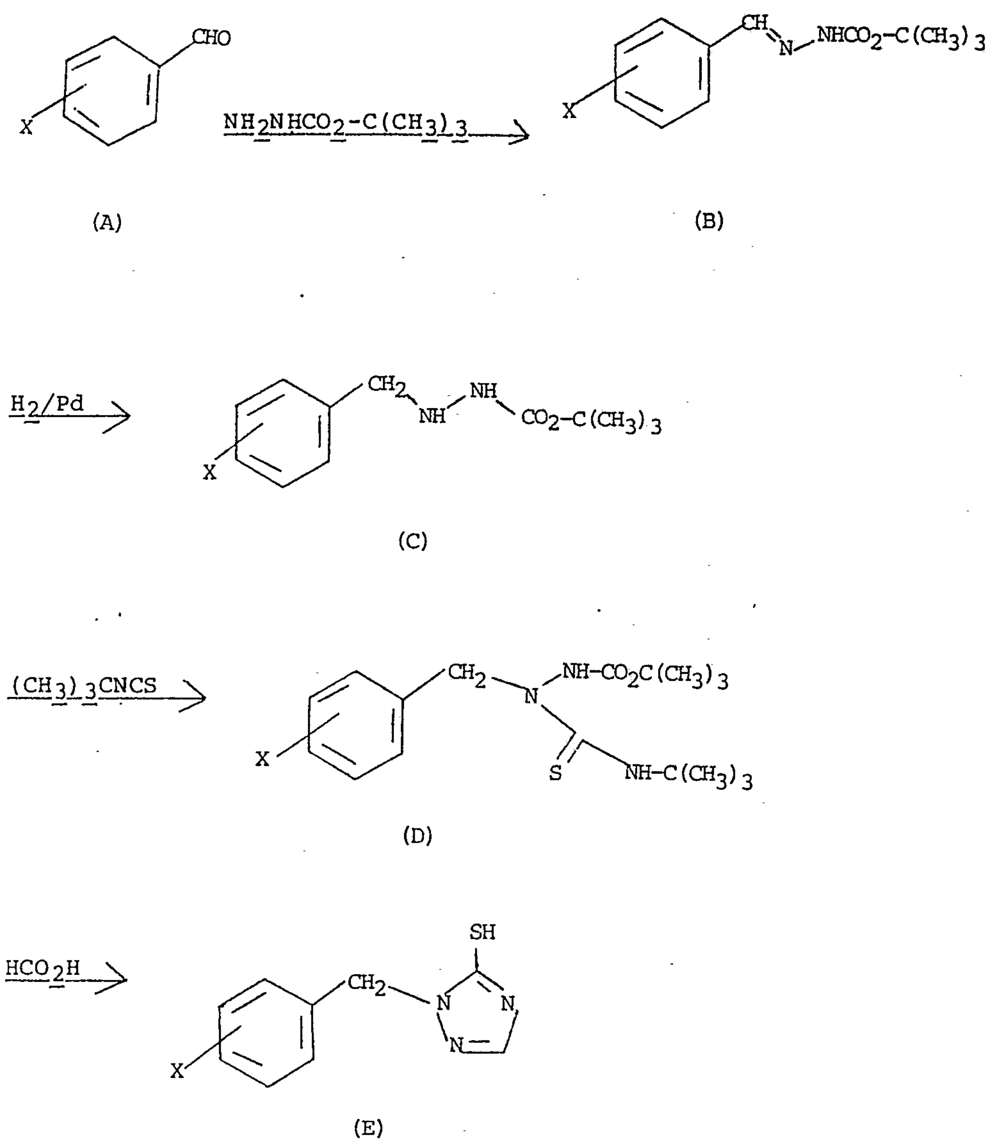


Dealkylation of the corresponding 1-alkoxyaralkyl substituted-1,2,4-triazole-5-thiol compounds of Formula (E) with boron tribromide or hydrobromic acid yields the 1-hydroxyaralkyl substituted-1,2,4-triazole-5-thiols of the present invention.

The compounds wherein R is a methyl group are prepared by alkylating corresponding 1-aralkyl substituted-1,2,4-triazole-5-thiols included within the Formula (E) compounds with methyl iodide in methanol by known procedures. Other alkyl halides such as methyl bromide or methyl chloride, in appropriate solvents, can be substituted for methyl iodide. Further, the compounds in which R is an alkyl group other than methyl are prepared by reacting the corresponding 1-aralkyl substituted-1,2,4-triazole-5-thiol compounds of Formula (E) with an alkyl halide, such as butyl iodide, in an appropriate solvent to yield the desired 1-aralkyl substituted-1,2,4-triazole-5-alkylthiol compound of the present invention.

As illustrated in Scheme I, n is 1; however, n can be from 0 to 5. The compounds wherein n is 0 preferably are prepared from substituted phenylhydrazines which are substituted for the t-butoxycarbonylhydrazines (C) in the Scheme I process. The starting substituted phenylhydrazines are known and can be prepared by known processes. The compounds in which n is 2,3,4, or 5 also are prepared by the Scheme I process, except that the benzaldehydes (A) are replaced by phenyl $C_{1-4}$alkylaldehydes such as phenethylaldehyde, 3-phenyl-1-propionaldehyde, or 4-phenyl-1-butyraldehyde.

In preparing the presently invented substituted 1-aralkyl-1,2,4-triazole-5-thiol and substituted 1-aralkyl-1,2,4-triazole-5-alkylthiol compounds, novel intermediate compounds of the following formula are synthesized:

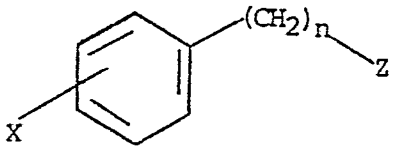

in which:
n is 0 to 4;
X is hydrogen, halo, $C_{1-4}$ alkyl, CN, $NO_2$, $SO_2NH_2$, COOH, CHO, OH, $CH_2OH$, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents; and
Z is $C{=}NNHCO_2C(CH_3)_3$, $CH_2NHNHCO_2C(CH_3)_3$, or

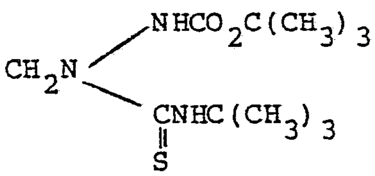

except compounds in which n is 0, Z is $C{=}NNHCO_2C(CH_3)_3$ or $CH_2NHNHCO_2C(CH_3)_3$ and X is H.

Novel intermediates of the following formula also are synthesized in preparing the presently invented DBH inhibitors:

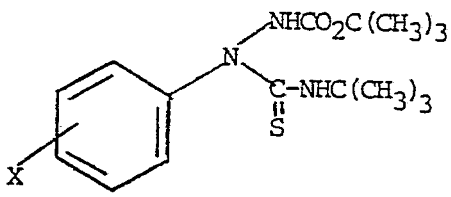

in which:
X is halo, $C_{1-4}$ alkyl, CN, $NO_2$, $SO_2NH_2$, COOH, CHO, OH, $CH_2OH$, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents.

The pharmaceutically acceptable acid addition salts of the compounds of the invention are formed with strong or moderately strong organic or inorganic acids by methods known to the art. For example, the base is reacted with an inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of the salts which are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts.

Because the compounds of Formulae I and II inhibit DBH activity, they have therapeutic value as diuretic, natriuretic, cardiotonic, antihypertensive and vasodilator agents, as well as antiulcerogenic agents. Listed in

Table I are the compounds of the invention and compounds included in the pharmaceutical compositions of the invention and useful in the methods of the invention that were tested for in vitro DBH inhibition by a standard procedure for assaying conversion of tyramine to octopamine in the presence of DBH. J. J. Pisano, et al., Biochim. Biophys. Acta, 43, 566-568 (1960). Octopamine was assayed following sodium periodate oxidation to p-hydroxybenzaldehyde by measuring spectrophotometric absorbance at 330 nm. In Table I, inhibition is given in molar concentration of compound at which DBH activity was halved ($IC_{50}$). Fusaric acid, by this test was found to have an $IC_{50}$ of $8 \times 10^{-7}$M.

Table I

| Compound | $IC_{50}$ |
|---|---|
| 1-benzyl-1,2,4-triazole-5-thiol | $7.5 \times 10^{-5}$ |
| 1-(3'-fluorobenzyl)-1,2,4-triazole-5-thiol | $1.4 \times 10^{-5}$ |
| 1-(3',5'-difluorobenzyl)-1,2,4-triazole-5-thiol | $5.8 \times 10^{-6}$ |

Further, spontaneously hypertensive rats were treated with a suspension or solution of 1-benzyl-1,2,4-triazole-5-thiol at a dose of 100 mg/kg orally, and mean arterial blood pressure was monitored for 260 minutes using indwelling cannulae positioned in the tail arteries. When compared to vehicle-treated controls, the compound-treated animals' blood pressure was approximately 5% below that of controls 20 minutes following treatment. The compound-treated animals' blood pressure continued to fall until it reached its nadir of approximately 20% less than controls at 240 minutes after compound treatment. Similarly, 1-(3'-fluorobenzyl)-1,2,4-triazole-5-thiol produced approximate 20% blood pressure reductions one hour after a 50 mg/kg intraperitoneal dose. The blood pressure of the treated animals remained approximately 20% less than controls when blood pressure monitoring was terminated.

The compounds of Formula II can be incorporated into convenient dosage forms such as capsules, tablets, or injectable preparations. Solid or liquid pharmaceutical carriers can be employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid such as an ampoule, or an aqueous or nonaqueous liquid suspension.

The pharmaceutical preparations are made following conventional techniques of a pharmaceutical chemist involving mixing, granulating and compressing, when necessary, for tablet forms, or mixing, filling and dissolving the ingredients, as appropriate, to give the desired oral or parenteral products.

Doses of the present compounds of Formula II in a pharmaceutical dosage unit as described above will be an efficacious, nontoxic quantity selected from the range of 0.1-100 mg/kg of active compound, preferably 0.1-50 mg/kg. The selected dose is administered to a human patient in need of DBH inhibition from 1-6 times daily, orally, rectally, by injection, or continuously by infusion. Oral dosage units for human administration preferably contain from 1 to 500 mg of active compound. Parenteral administration, which uses lower dosages is preferred. Oral administration, at higher dosages, however, also can be used when safe and convenient for the patient.

The method of this invention of inhibiting DBH activity in mammals, including humans, comprises administering internally to a subject in need of such inhibition an effective DBH inhibiting amount of a compound of Formula II.

The following examples are illustrative of preparation of Formula II compounds. The examples are not intended to limit the scope of the invention as defined hereinabove and as claimed below.

EXAMPLE 1

1-(3'-Fluorobenzyl)-1,2,4-triazole-5-thiol

A solution of 3-fluorobenzaldehyde (6.21 g, 0.05 mole) and t-butylcarbazate (6.61 g, 0.05 mole) in hexane (50 ml) was heated under reflux for 30 minutes. The mixture was cooled in ice and the product filtered and recrystallized from ethanol to give 1-(3'-fluorobenzaldehyde)-t-butoxycarbonylhydrazone, m.p.: 165-168°C (8.79 g, 74%).

A mixture of 1-(3'-fluorobenzaldehyde)-t-butoxycarbonylhydrazone (1.02 g, 4.28 mmole) and 10% palladium on carbon (220 mg) in methanol (40 ml) was shaken under hydrogen (40 lbs.) for 1 hour. The reaction mixture

was filtered and the solvent removed under vacuum to give 1-(3′-fluorobenzyl)-2-t-butoxycarbonylhydrazine (1.00 g, 97%).

A solution of 1-(3′-fluorobenzyl)-2-t-butoxycarbonylhydrazine (2.29 g, 9.53 mmole) and t-butylisothiocyanate (2.41 ml., 19.1 mmole) in ethyl acetate (15 ml) was refluxed for 7 hours. The solvent was removed under vacuum and the residue was triturated with hexane and the product was filtered and dried to yield 1-t-butoxycarbonyl-2-(3′-fluorobenzyl)-4-t-butylthiosemicarbazide (1.28 g, 38%).

A solution of 1-t-butoxycarbonyl-2-(3′-fluorobenzyl)-4-t-butylthiosemicarbazide (1.28 g, 3.60 mmole) in 98% formic acid (15 ml) was refluxed for 4 hours and the solvent was removed under vacuum. The residue was dissolved in methylene chloride-ethyl acetate (80:20) and purified by flash chromatography (silica) to give 1-(3′-fluorobenzyl)-1,2,4-triazole-5-thiol, m.p.: 155-156°C (165 mg, 22%).

EXAMPLE 2

1-(3′,5′-Difluorobenzyl)-1,2,4-triazole-5-thiol

A mixture of 3,5-difluorobenzonitrile (5.0 g, 0.0359 mole) and Raney catalyst powder in 90% formic acid (50 ml) was stirred under reflux for 2 hours and the catalyst filtered and washed with hot water and hexane. The hexane layer was separated and the aqueous solution was extracted an additional four times with hexane. The combined hexane extracts were washed with water and brine, dried and the solvent was removed to give 3,5-difluorobenzaldehyde (3.37 g, 66%).

A solution of 3,5-difluorobenzaldehyde (3.37 g, 0.0237 mole) and t-butylcarbazate (3.13 g, 0.0237 mole) in hexane (60 ml) was heated under reflux for 30 minutes. The mixture was cooled in ice and the product filtered and recrystallized from ethanol to give 1-(3′,5′-difluorobenzaldehyde)-2-t-butoxycarbonylhydrazone, m.p.: 190-191°C (3.52 g, 58%).

A mixture of 1-(3′,5′-difluorobenzaldehyde)-t-butoxycarbonylhydrazone (3.49 g, 13.6 mmole) and 10% palladium on carbon (300 mg) in methanol (50 ml) was shaken under hydrogen (42 lbs.) for 4.5 hours and the catalyst removed by filtration. The solution was charged again with 10% palladium on carbon (400 mg) and shaken under hydrogen for 1.5 hours. The reaction mixture was filtered and the solvent removed under vacuum to give 1-(3′,5′-difluorobenzyl)-2-t-butoxycarbonylhydrazine (3.34 g, 95%).

A solution of 1-(3′,5′-difluorobenzyl)-2-t-butoxycarbonylhydrazine (3.22 g, 12.5 mmole) and t-butylisothiocyanate (1.6 ml, 12.6 mmole) in ethyl acetate (30 ml) was refluxed for 17 hours. An additional 2 ml of t-butylisothiocyanate was added and the mixture was refluxed for 5 hours. The solvent was removed under vacuum and the residue was triturated with hexane and the product was filtered and dried to yield 1-t-butoxycarbonyl-2-(3′,5′- difluorobenzyl)-4-t-butylthiosemicarbazide (3.03 g, 65%).

A solution of 1-t-butoxycarbonyl-2-(3′,5′-difluorobenzyl)-4-t-butylthiosemicarbazide (2.98 g, 7.98 mmole) in 98% formic acid (40 ml) was refluxed for 4 hours and the solvent was removed under vacuum. The residue was triturated with ether and the resulting solid was filtered. The solid was dissolved in methylene chloride-ethyl acetate (80:20) and purified by flash chromatography (silica) to give 1-(3′,5′-difluorobenzyl)-1,2,4-triazole-5-thiol, m.p.: 188-189°C (407 mg, 22%).

EXAMPLE 3

1-Benzyl-1,2,4-triazole-5-thiol

The process of Example 1, beginning with benzaldehyde in place of 3-fluorobenzaldehyde yields 1-benzyl-1,2,4-triazole-5-thiol

EXAMPLE 4

1-(3′,5′-Difluorobenzyl)-5-methylthio-1,2,4-triazole

The reaction of 1-(3′,5′-difluorobenzyl)-1,2,4-triazole-5-thiol (prepared as in Example 2) with methyl iodide and sodium methoxide in methanol by known techniques yields (1-(3′,5′-difluorobenzyl)-5-methylthio-1,2,4-triazole.

EXAMPLE 5

1-(3′-Fluorophenyl)-1,2,4-trizole-5-thiol

The process of Example 1, wherein 1-(3′-fluorobenzyl)-2-t-butoxycarbonylhydrazine is replaced by 3-fluorophenylhydrazine yields 1-(3′-fluorophenyl)-1,2,4-triazole-5-thiol.

EXAMPLE 6

1-(3′-Phenyl-1′-propyl)-1,2,4-triazole-5-thiol

The process of Example 1 beginning with 3-phenyl-1-propionaldehyde in place of 3-fluorobenzaldehyde yields 1-(3′-phenyl-1′-propyl)-1,2,4-triazole-5-thiol.

EXAMPLE 7

1-(3'-Cyanobenzyl)-1,2,4-triazole-5-thiol

The process of Example 1 beginning with 3-cyano-benzaldehyde in place of 3-fluorobenzaldehyde yields 1-(3'-cyanobenzyl)-1,2,4-triazole-5-thiol.

EXAMPLE 8

1-(3'-Nitrophenyl)-1,2,4-triazole-5-thiol

The process of Example 1 beginning with 3-nitrophenylhydrazine in place of 1-(3'-fluorobenzyl)-2-t-butoxycarbonylhydrazine yields 1-(3'-nitrophenyl)-1,2,4-triazole-5-thiol.

EXAMPLE 9

1-(4'-Methylsulfonylbenzyl-1,2,4-triazole-5-thiol

The process of Example 1 beginning with 4-methyl-sulfonylbenzaldehyde in place of 3-fluorobenzaldehyde yields 1-(4'-methylsulfonylbenzyl)-1,2,4-triazole-5-thiol.

EXAMPLE 10

1-(4'-Carboethyoxybenzyl)-1,2,4-triazole-5-thiol

The process of Example 1 beginning with 4-carboethoxybenzaldehyde in place of 3-fluorobenzaldehyde yields 1-(4'-carboethoxybenzyl)-1,2,4-triazole-5-thiol.

EXAMPLE 11

An oral dosage form for administering the presently invented compounds is produced by screening, mixing, and filling into hard gelatin capsules the ingredients in the proportions shown in Table III, below.

Table III

| Ingredients | Amounts |
|---|---|
| 1-(3'-Fluorobenzyl)-1,2,4-triazole-5-thiol | 50 mg |
| magnesium stearate | 5 mg |
| lactose | 75 mg |

EXAMPLE 12

The sucrose, calcium sulfate dihydrate and 1-aralkyl substituted-1,2,4-triazole-5-thiol shown in Table IV below, are mixed and granulated in the proportions shown with a 10% gelatin solution. The wet granules are screened, dried, mixed with the starch, talc and stearic acid, screened and compressed into a tablet.

Table IV

| Ingredients | Amounts |
|---|---|
| 1-(3',5'-Difluorobenzyl)-1,2,4-triazole-5-thiol | 100 mg |
| calcium sulfate dihydrate | 150 mg |
| sucrose | 20 mg |
| starch | 10 mg |
| talc | 5 mg |
| stearic acid | 3 mg |

EXAMPLE 13

1-(3′-5′-Difluoro-4′-hydroxybenzyl)-1,2,4-triazole-5-thiol hydrochloride, 75 mg, is dispursed in 25 ml of normal saline to prepare an injectable preparation.

While the preferred embodiments of the invention are illustrated by the above, it is to be understood that the invention is not limited to the precise instructions herein disclosed and that the right to all modifications coming within the scope of the following claims is reserved.

**Claims**

Claims for the Following Contracting States: BE, CH, DE, FR, GB, IT, LI, LU, NL and SE

1. A compound represented by the Formula (I) :

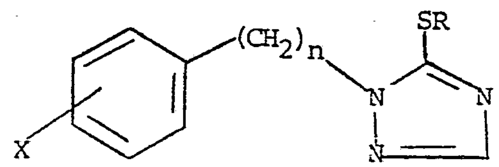

(I)

in which:
n is 0-5;
R is hydrogen or $C_{1-4}$ alkyl; and
X is hydrogen halo, $C_{1-4}$ alkyl, CN, $NO_2$, $SO_2NH_2$, COOH, CHO $CH_2OH$, OH, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents, or any pharmaceutically acceptable salt or hydrate thereof; except compounds in which:
n is 0 or 1 and X is hydrogen.

2. A compound according to Claim 1 in which R is hydrogen.

3. A compound according to Claim 2 in which n is 1.

4. The compound according to Claim 3 that is 1-(3′-fluorobenzyl)-1,2,4-triazole-5-thiol or 1-(3′,5′-difluorobenzyl)-1,2,4-triazole-5-thiol.

5. A pharmaceutical composition comprising a compound having the Formula (II) :

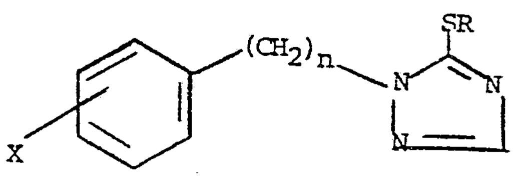

(II)

in which:
n is 0-5;

R is hydrogen or $C_{1-4}$ alkyl; and
X is hydrogen, halo, $C_{1-4}$ alkyl, CN, $NO_2$, $SO_2NH_2$, COOH, CHO, $CH_2OH$, OH, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to five substituents; or any pharmaceutically acceptable salt or hydrate thereof; and
a suitable pharmaceutical carrier.

6. A composition of Claim 5 in which the compound is 1-benzyl-1,2,4-triazole-5-thiol, 1-(3′-fluorobenzyl)-1,2,4-triazole-5-thiol, or 1-(3′5′-difluorobenzyl)-1,2,4- triazole-5-thiol.

7. A compound having the Formula:

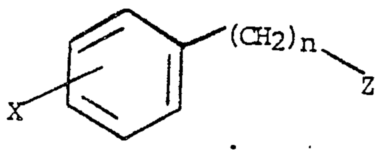

in which:
n is 0 to 4;
X is hydrogen, halo, $C_{1-4}$ alkyl, CN, $NO_2$, $SO_2NH_2$, COOH, CHO, OH, $CH_2OH$, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents; and
Z is $C{=}NNHCO_2C(CH_3)_3$, $CH_2NHNHCO_2C(CH_3)_3$, or

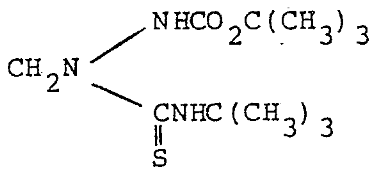

except compounds in which n is 0, X is H, and Z is $C{=}NNHCO_2C(CH_3)_3$ or $CH_2NHNHCO_2C(CH_3)_3$.

8. The compound of Claim 7 that is 1-(3′-fluorobenzaldehyde)-t-butoxycarbonylhydrazone, 1-(3′-fluorobenzyl)-2-t-butoxycarbonylhydrazine, 1-t-butoxycarbonyl-2-(3′-fluorobenzyl)-4-t-butylthiosemicarbazide, 1-(3′,5′-difluorobenzaldehyde)-t-butoxycarbonylhydrazone, 1-(3′,5′-difluorobenzyl)-2-t-butoxycarbonylhydrazine, 1-t-butoxycarbonyl-2-(3′,5′-fluorobenzyl)-4-t-butylthiosemicarbazide, or 1-t-butoxycarbonyl-2-benzyl-4-t-butylthiosemicarbazide.

9. A compound having the formula:

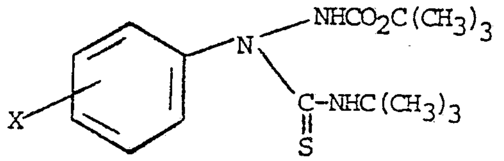

in which:
X is halo, $C_{1-4}$ alkyl, CN, $NO_2$, $SO_2NH_2$, COOH, CHO, OH, $CH_2OH$, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents.

10. A process for preparing compounds of Formula:

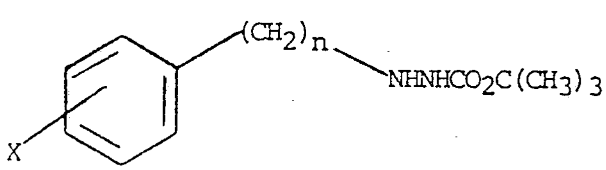

in which:
X is hydrogen, halo, $C_{1-4}$ alkyl, CN, $NO_2$, $SO_2NH_2$, COOH, CHO, $CH_2OH$, OH, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents; and
n is 1-5;
or any pharmaceutically acceptable salt or hydrate thereof that comprises hydrogenation of a compound of the formula:

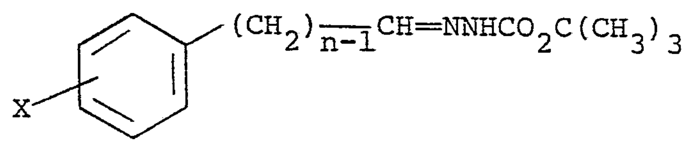

in which:
X and n are as defined hereinabove and thereafter optionally forming a pharmaceutically acceptable salt or hydrate thereof.

11. The process of Claim 10 wherein the compound prepared is 1-benzyl-2-t-butoxycarbonylhydrazine, 1-(3′-fluorobenzyl)-2-t-butoxycarbonylhydrazine, or 1-(3′,5′-difluorobenzyl)-2-t-butoxycarbonylhydrazine.

12. A process for preparing compounds of Formula

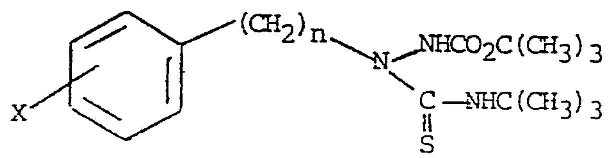

in which:
n is 1-5;
X is hydrogen, halo, $C_{1-4}$ alkyl, CN, $NO_2$, $SO_2NH_2$, COOH, CHO, $CH_2OH$, OH, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents, or any pharmaceutically acceptable salt or hydrate thereof that comprises:
reacting in an inert organic solvent t-butylisothiocyanate with a compound of the formula:

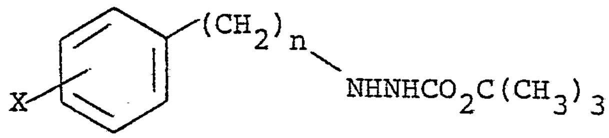

in which n and X are as defined hereinabove and thereafter optionally forming a pharmaceutically acceptable salt or hydrate thereof.

13. The process of Claim 12 wherein the compound prepared is 1-t-butoxycarbonyl-2-benzyl-4-t-butylthiosemicarbazide, 1-t-butoxycarbonyl-2-(3′-fluorobenzyl-4-t-butylthiosemicarbazide, or 1-t-butoxycarbonyl-2-(3′, 5′-difluorobenzyl)-4-t-butylthiosemicarbazide.

14. A process for preparing compounds of Formula (II) :

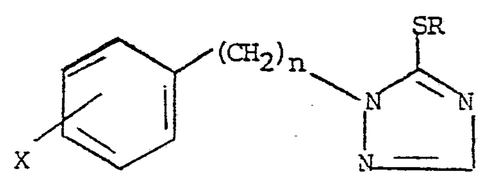

(II)

in which:
n is 0-5;
X is hydrogen, halo, $C_{1-4}$ alkyl, CN, $NO_2$, $SO_2NH_2$, COOH, CHO, $CH_2OH$, OH, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents; and
R is hydrogen or $C_{1-4}$ alkyl; or any pharmaceutically acceptable salt or hydrate thereof that comprises:
cyclizing in the presence of strong acid a compound of the Formula:

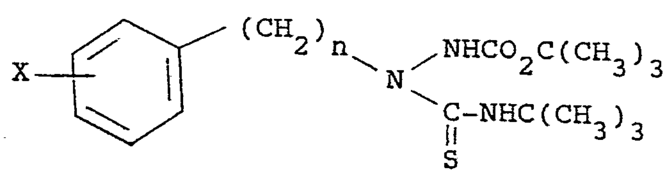

in which :
X and n are as in Formula (II), and when R is $C_{1-4}$ alkyl, alkylating a Formula (II) compound and thereafter

optionally forming a pharmaceutically acceptable salt or hydrate thereof.

15. A compound of Claim 1 for use as a therapeutic agent.

16. A compound of Formula (II) as defined in Claim 14 for use as a dopamine-β-hydroxylase inhibitor.

17. Use of compounds of the formula :

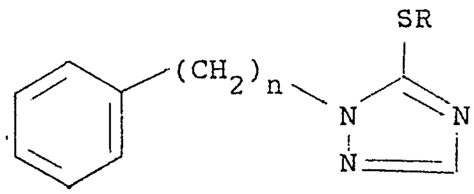

in which :
n is 0 or 1; and
R is hydrogen or $C_{1-4}$alkyl in the preparation of an agent for the treatment of hypertension.

Claims for the Following Contracting States : AT, GR and ES

1. A process for preparing compounds of Formula (II) :

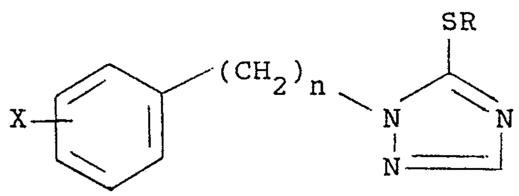

(II)

in which :
n is 0-5;
X is hydrogen, halo, $C_{1-4}$alkyl, CN, $NO_2$, $SO_2NH_2$, COOH, CHO, $CH_2OH$, OH, $C_{1-4}$alkoxy, $CF_3$, $SO_2CH_3$, $SO_2CF_3$, or $CO_2C_aH_{2a+1}$ wherein a is 1-5 or any synthetically accessible combination thereof of up to 5 substituents; and
R is hydrogen or $C_{1-4}$alkyl;
or any pharmaceutically acceptable salt or hydrate thereof that comprises :
cyclizing in the presence of strong acid a compound of the Formula :

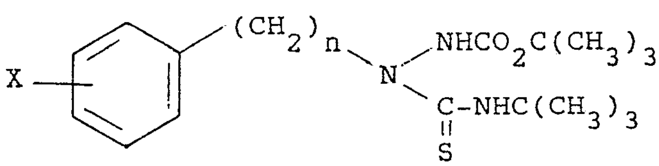

in which :
X and n are as in Formula (II), and when R is $C_{1-4}$ alkyl, alkylating a Formula (II) compound and thereafter optionally forming a pharmaceutically acceptable salt or hydrate thereof.

2. A process according to Claim 1 in which the acid is formic acid.

3. The process of Claim 1 or 2 wherein the compound prepared is 1-benzyl-1,2,4-triazole-5-thiol.

4. The process of Claim 1 or 2 wherein the compound prepared is 1-(3′-fluorobenzyl)-1,2,4-triazole-5-thiol.

5. The process of Claim 1 or 2 wherein the compound prepared is 1-(3′, 5′-difluorobenzyl)-1,2,4-triazole-5-thiol.

6. A process for preparing compounds of Formula :

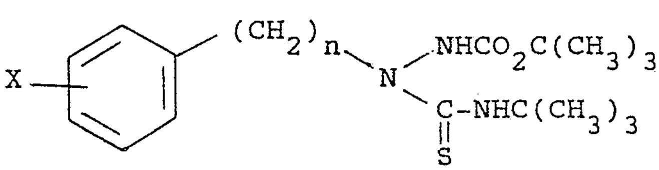

in which :
n is 1-5;
X is hydrogen, halo, $C_{1-4}$ alkyl, CN, $NO_2$, $SO_2NH_2$, COOH, CHO, $CH_2OH$, OH, $C_{1-4}$alkoxy, $CF_3$, $SO_2CH_3$,

$SO_2CF_3$, or $CO_2C_aH_{2a+1}$ wherein a is 1-5, or any synthetically accessible combination thereof of up to 5 substituents, or any pharmaceutically acceptable salt or hydrate thereof that comprises : reacting in an inert organic solvent t-butylisothiocyanate with a compound of the formula :

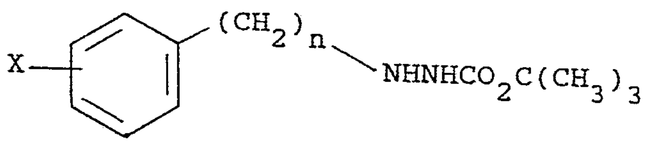

in which n and X are as defined hereinabove and thereafter optionally forming a pharmaceutically acceptable salt or hydrate thereof.

7. A process according to claim 6 in which the inert organic solvent is ethyl acetate.

8. The process of Claim 6 or 7 wherein the compound prepared is 1-t-butoxycarbonyl-2-benzyl-4-t-butylthiosemicarbazide, 1-t-butoxycarbonyl-2-(3′-fluorobenzyl)-4-t-butylthiosemicarbazide, or 1-t-butoxycarbonyl-2-(3′,5′-difluorobenzyl)-4-t-butylthiosemicarbazide.

9. A process for preparing compounds of Formula

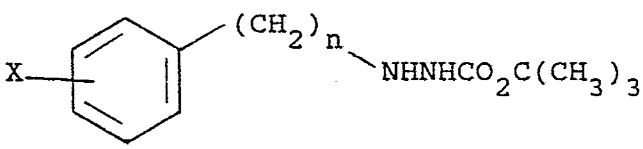

in which:
X is hydrogen, halo, $C_{1-4}$alkyl, CN, $NO_2$, $SO_2NH_2$, COOH, CHO, $CH_2OH$, OH, $C_{1-4}$ alkoxy, $CF_3$, $SO_2CH_3$, $SO_2CF_3$, or $CO_2C_aH_{2a+1}$ wherein a is 1-5, or any synthetically accessible combination thereof of up to 5 substituents; and
n is 1-5;
or any pharmaceutically acceptable salt or hydrate thereof that comprises hydrogenation of a compound of the Formula :

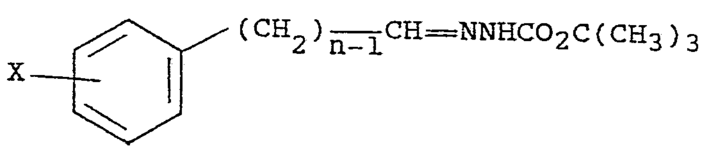

in which :
X and n are as defined hereinabove and thereafter optionally forming a pharmaceutically acceptable salt or hydrate thereof.

10. A process according to claim 9 in which hydrogenation is carried out with palladium on carbon in the presence of methanol.

11. A process of Claim 9 or 10 wherein the compound prepared in 1-benzyl-2-t-butoxycarbonylhydrazine, 1-(3′-fluorobenzyl)-2-t-butoxycarbonylhydrazine, or 1-(3′,5′-difluorobenzyl-2-t-butoxycarbonylhydrazine.

12. A process for the preparation of a pharmaceutical composition which comprises bringing into association a compound of Formula (II) as defined in Claim 1 and a pharmaceutically acceptable carrier.

13. A pharmaceutical composition according to Claim 12 in which the compound of Formula (II) is : 1-benzyl-1,2,4-triazole-5-thiol, 1-(3′-fluorobenzyl)-1,2,4-triazole-5-thiol, or 1-(3′,5′-difluorobenzyl)-1,2,4-triazole-5-thiol.